# EUROPÄISCHE PATENTANMELDUNG

(11) **EP 4 015 495 A1**
(43) Veröffentlichungstag der Anmeldung: **22.06.2022**
(21) Anmeldenummer: 20215531.3
(22) Anmeldetag: 18.12.2020
(51) Int. Cl.: C07C 5/48, C07C 45/50, C07C 11/04, C07C 47/02, C07C 11/06, C07C 31/10, C07C 1/24, C07C 29/141

(54) **VERFAHREN UND ANLAGE ZUR HERSTELLUNG EINER ZIELVERBINDUNG**

(71) Anmelder: Linde GmbH, 82049 Pullach (DE)
(72) Erfinder: Meiswinkel, Andreas, 83253 Rimsting (DE); Schubert, Martin, 81375 München (DE); Winkler, Florian, 81241 München (DE); De Val, Ricardo Bermejo, 80807 München (DE); Haidegger, Ernst, 85521 Riemerling (DE)
(74) Vertreter: DehnsGermany Partnerschaft von Patentanwälten

(57) **Zusammenfassung**

Es wird ein Verfahren (100-500) zur Herstellung einer Zielverbindung vorgeschlagen, bei dem ein erstes Komponentengemisch unter Erhalt eines zweiten Komponentengemischs einer oxidativen Dehydrierung (10) unterworfen wird, und bei dem zumindest ein Teil des zweiten Komponentengemischs unter Erhalt eines dritten Komponentengemischs einer Hydroformylierung (20) unterworfen wird, wobei das erste Komponentengemisch ein Paraffin, das zweite Komponentengemisch ein in der oxidativen Dehydrierung (10) aus dem Paraffin gebildetes Olefin und das dritte Komponentengemisch eine in der Hydroformylierung (20) aus dem Olefin gebildete Folgeverbindung, die die Zielverbindung oder eine zu der Zielverbindung umsetzbare Vorläuferverbindung darstellt, enthalten. Das erste Komponentengemisch weist Sauerstoff in einem Sauerstoffgehalt und/oder Kohlendioxid in einem Kohlendioxidgehalt auf, und das zweite Komponentengemisch weist Kohlenmonoxid in einem Kohlenmonoxidgehalt und das Olefin in einem Olefingehalt auf, wobei der Kohlenmonoxidgehalt bei 5% bis 120% des Olefingehalts beträgt. Eine entsprechende Anlage (100-500) ist ebenfalls Gegenstand der Erfindung.

## Beschreibung

Die vorliegende Erfindung betrifft ein Verfahren und eine Anlage zur Herstellung einer Zielverbindung gemäß den Oberbegriffen der entsprechenden unabhängigen Patentansprüche.

### Hintergrund der Erfindung

Die Herstellung von Propylen (Propen) ist in der Fachliteratur, beispielsweise in dem Artikel "Propylene" in Ullmann's Encyclopedia of Industrial Chemistry, Ausgabe 2012, beschrieben. Propylen wird herkömmlicherweise durch Dampfspalten (Steam Cracking) von Kohlenwasserstoffeinsätzen und Umwandlungsverfahren im Zuge von Raffinerieprozessen hergestellt. In letzteren Verfahren wird Propylen nicht notwendigerweise in der gewünschten Menge und nur als eine von mehreren Komponenten in einem Gemisch mit weiteren Verbindungen gebildet. Andere Verfahren zur Herstellung von Propylen sind ebenfalls bekannt, aber nicht in allen Fällen, beispielsweise hinsichtlich Effizienz und Ausbeute, zufriedenstellend.

Für die Zukunft wird ein deutlich steigender Bedarf und ggf. sogar Mangel an Propylen prognostiziert ("Propylen-Gap"), der die Bereitstellung entsprechend verbesserter Verfahren erfordert. Gleichzeitig gilt es, Kohlendioxidemissionen zu reduzieren oder ganz zu verhindern. Als potentielle Ausgangsverbindung stehen andererseits große Mengen an Methan bereit, die derzeit nur sehr begrenzt einer stofflichen Verwertung zugeführt und überwiegend verbrannt werden. In entsprechenden Erdgasfraktionen sind zudem oft nennenswerte Mengen an Ethan vorhanden.

Grundsätzlich existiert neben den zuvor erwähnten Dampfspaltverfahren eine Vielzahl von unterschiedlichen Verfahren zur Umwandlung von Kohlenwasserstoffen und verwandten Verbindungen ineinander, von denen nachfolgend einige genannt und weiter unten detaillierter erläutert werden.

Die Herstellung von Propylen aus Propan durch Dehydrierung (PDH) stellt ein kommerziell verfügbares und etabliertes Verfahren dar. Ebendies gilt für die Herstellung von Propylen aus Ethylen durch die Olefinmetathese. Dieses Verfahren erfordert 2-Buten als zusätzliches Edukt.

Ferner existieren sogenannte Methane-to-Olefine- bzw. Methane-to-Propylene-Verfahren (MTO, MTP), in denen aus Methan zunächst Synthesegas hergestellt wird und das Synthesegas anschließend zu Olefinen wie Ethylen und Propylen umgesetzt wird. Entsprechende Verfahren können auf Grundlage von Methan, jedoch auch auf Grundlage von anderen Kohlenwasserstoffen oder kohlenstoffhaltigen Ausgangsmaterialien wie Kohle oder Biomasse betrieben werden.

Die vorliegende Erfindung stellt sich die Aufgabe, ein verbessertes Verfahren zur Herstellung insbesondere von Propylen als Zielverbindung, jedoch auch zur Herstellung anderer organischer Zielverbindungen, insbesondere von Oxoverbindungen wie Aldehyden und Alkoholen mit einem entsprechenden Kohlenstoffgrundgerüst, bereitzustellen.

### Offenbarung der Erfindung

Die vorstehend genannte Aufgabe wird durch ein Verfahren und eine Anlage zur Herstellung einer Zielverbindung mit den Merkmalen der jeweiligen unabhängigen Patentansprüche gelöst. Bevorzugte Ausgestaltungen sind jeweils Gegenstand der abhängigen Patentansprüche und der nachfolgenden Beschreibung.

Vor der Erläuterung der Merkmale und Vorteile der vorliegenden Erfindung werden zunächst noch weitere Grundlagen der Erfindung näher erläutert und bei der Beschreibung der Erfindung verwendete Begriffe definiert.

Die Erfindung geht insbesondere von der oxidativen Dehydrierung von Ethan (ODHE) aus, die aus der Literatur bekannt ist, aber kommerziell noch nicht eingesetzt wurde. Verschiedene Katalysatorsysteme wurden auf ihre Eignung bzgl. der ODHE untersucht (vgl. u.a. C.A. Gärtner et al., "Oxidative Dehydrogenation of Ethane: Common Principles and Mechanistic Aspects", ChemCatChem, 2013, 5, 3196-3217). Die ODHE stellt eine Alternative zum nicht-katalytischen Dampfspalten von Ethan dar, bei dem Ethylen das kommerzielle Hauptprodukt darstellt, wie erläutert.

Die vorliegende Erfindung ist grundsätzlich jedoch nicht auf die oxidative Dehydrierung von Ethan beschränkt, sondern kann sich auch auf die oxidative Dehydrierung (ODH) anderer Paraffine wie Propan oder Butan erstrecken. Die nachfolgenden Erläuterungen gelten in diesem Fall entsprechend.

Insbesondere haben sich für die ODHE MoVNb-basierte Katalysatorsysteme als besonders vielversprechend herausgestellt (vgl. F. Cavani et al., "Oxidative dehydrogenation of ethane and propane: How far from commercial implementation?", Catal. Today, 2007, 127, 113-131). Auch zusätzlich Te enthaltende Katalysatorsysteme können verwendet werden. Ist hier von einem "MoVNb-basierten Katalysatorsystem" oder einem "MoVTeNb-basierten Katalysatorsystem" die Rede, sei hierunter ein Katalysatorsystem verstanden, das die genannten Elemente als Mischoxid aufweist, auch ausgedrückt mit MoVNbOₓ bzw. MoVTeNbOₓ. Die Erfindung kommt insbesondere mit solchen Katalysatorsystemen zum Einsatz.

Bei der ODHE wird unter industriellen Einsatzbedingungen und unter Einsatz der genannten Katalysatorsysteme auch Essigsäure in signifikantem Maße koproduziert, welche ebenfalls als kommerzielles Produkt wirtschaftlich genutzt werden kann.

Abhängig von den Reaktionsbedingungen und verwendeten Katalysatoren entstehen in der ODHE gewisse Mengen an Kohlenmonoxid und Kohlendioxid als (herkömmlicherweise als unerwünscht betrachtete) Nebenprodukte. Herkömmliche Verbesserungs- und Optimierungsansätze sind entsprechend darauf ausgerichtet, den Anteil an diesen Kohlenstoffoxiden zu minimieren. Aktuell kann der Anteil an den genannten Kohlenstoffoxiden auf weniger als 5% zurückgedrängt werden.

Neuerdings sind kommerzielle Konzepte bekannt geworden, die eben insbesondere auf den genannten Mischoxidkatalysatoren beruhen (siehe hierzu insbesondere WO 2018/115416 A1, WO 2018/115418 A1, WO 2018/082945 A1, US 10,730,810 B2, WO 2018/115494 A1, WO 2019/243480 A1, US 10,730,811 B2, WO 2018/153831 A1, WO 2018/019761 A1, WO 2018/019760 A1, WO 2017/198762 A1, WO 2017/144584 A1, WO2020074750A1, US 9,963,412 B2 und US 10,017,432 B) und allesamt Luft bzw. Sauerstoff als Oxidationsmittel verwenden und nur untergeordnete Mengen an Kohlenstoffoxiden im Produktstrom enthalten (sollen).

Im Allgemeinen finden auch bei anderen technisch relevanten oxidativen Prozessen wie z.B. der Herstellung von Maleinsäureanhydrid (MSA) aus Butan, Buten oder Benzol oder auch der zweistufigen Synthese von Acrylsäure aus Propylen über das Intermediat Acrolein sauerstoffarme Bedingungen Verwendung. Es erfolgt üblicherweise Verwendung von sauerstoffabgereicherter Luft als Oxidationsmittel. Beispielhaft sei hier die DE 198 37 519 A1 zur Oxidation von Propan zu Acrolein und/oder Acrylsäure genannt. Eine aktuelle Übersicht zur MSA-Synthese, die ausnahmslos mit Luft als Oxidationsmittel erfolgt, findet sich beispielsweise auch bei P.V. Mangili et al., "Eco-efficiency and techno-economic analysis for maleic anhydride manufacturing processes", Clean Technol. Environ. Policy 2019, 21,1073-1090.

Grundsätzlich wird im Rahmen einer Prozessintensivierung aber auch die Verwendung von Luft oder sauerstoffangereicherter Luft als Oxidationsmittel diskutiert. Nicht zuletzt spielt in diesem Zusammenhang aber immer auch die Vermeidung von explosionsfähigen Gemischen eine Rolle. Auch aus diesem Grund wird vorzugsweise Luft als Oxidationsmittel verwendet.

Die Einbringung von Stickstoff mit Luft oder sauerstoffan- oder -abgereicherter Luft erfordert in der Produktaufreinigung und -abtrennung eine Stickstoffabtrennung. Eine solche Stickstoffabtrennung ist zwar insbesondere für schwerere Oxygenate bzw. Kohlenwasserstoffe in diesen Prozessen technisch relativ problemlos realisierbar, aufgrund des niedrigen Siedepunktes von Stickstoff oftmals und insbesondere im Fall der ODHE aber nur schwierig bzw. aufwendig zu implementieren. Der Aufwand wird dabei entsprechend umso höher, je niedriger siedend das gewünschte Zielprodukt (bei der ODHE eben Ethylen) ist. Im Gegensatz zu MSA und Acrylsäure kann Ethylen nur unter kryogenen Bedingungen auskondensiert werden. Die Verwendung von Sauerstoff als Oxidationsmittel, insbesondere bei wenig verdünnten Bedingungen, führt dann jedoch zu explosionsfähigen Gemischen innerhalb der Anlage.

Entsprechende Mischkonzepte zur Vermeidung von explosionsfähigen Gemischen in bestimmten Anlagenteilen bzw. Reaktorbereichen sind zwar bekannt und z.B. in der EP 3 476 471 A1 für einen kommerziellen Rohrbündelreaktor beschrieben; diese oder andere Ansätze setzen jedoch neben konstruktiven Maßnahmen (z.B. dem Einbau von Flamm- oder Detonationssperren, der Minimierung von freien Volumina, druckfester Auslegung) insbesondere voraus, dass vor dem eigentlichen Reaktor die entsprechende Zündtemperatur deutlich unterschritten wird.

Des Weiteren ist aus der Literatur die Verwendung von Kohlendioxid als Oxidationsmittel bekannt, siehe beispielsweise US 2010/087615 A1, CA 2561986 A1, US 2,604,495 A und US 6,037,511 A sowie insbesondere L. Liu, et al., "Recent Advances on the Catalyst for Activation of CO2 in Several Typical Processes", in: New and Future Developments in Catalysis, Kapitel 7, Elsevier, 2013, 189-222.

Wird in der ODHE Kohlendioxid als Oxidationsmittel verwendet, werden in einer idealisierten Reaktion Ethylen und Kohlenmonoxid in einem stöchiometrischen Verhältnis von 1 zu 1 gebildet:

C₂H₆ + CO₂ → C₂H₄ + CO + H₂O

Als Nebenreaktionen werden dabei genannt:

C₂H₆ → C₂H₄ + H₂

(Dehydrierung)

C₂H₆ + 2 CO₂ → 4 CO + 3 H₂

(Reformierung, Reforming)

H₂ + CO₂ → CO + H₂ O

(Umgekehrte bzw. reverse Wassergasshift, RWGS)

In dem soeben zitierten Artikel werden diverse Katalysatoren aufgeführt, insbesondere basierend auf Na₂WO₄Mn, KCrMn, MnO, Ga₂O₃, Cr₂O₃, V₂O₅, CeO₂, CaOCeO₂, Mo₂C, Cr, K-Cr, CrMn, CrMnNi, CrMnLa, FeMn, NiO, CoBaCO₃, FeCr₂O₃, NiCr₂ O₃, MnCr₂O₃, CoCr₂O₃, CrCe und weitere in Kombination mit geeigneten Trägermaterialien. Kohlendioxid wird zumeist im Überschuss zugeführt und es werden teils Ethanumsätze von über 50% bis zu über 80% beschrieben, während die erzielten Ethylenausbeuten zwischen 8% und 66% liegen. Zur Ausbeute an Kohlenmonoxid werden in dem genannten Artikel hingegen keine Ausführungen gemacht.

Die zuletzt genannten Katalysatorsysteme wurden durchweg bei sehr hohen Temperaturen von über 550 °C, meist über 650 °C bis hin zu 800 °C getestet, um entsprechende Umsätze bzw. Ausbeuten zu erzielen. Bei solch hohen Temperaturen hat erwartungsgemäß die endotherme thermische Dehydrierung - die auch Grundlage für das Dampfspalten ist - bereits einen signifikanten Anteil am Reaktionsgeschehen.

Weitere Katalysatorsysteme und Details zu einer ODHE mit Kohlendioxid als Oxidationsmittel sind einem aktuellen Review-Artikel von Y. Gao et al., "Recent Advances in Intensified Ethylene Production - A Review", ACS Catalysis 2019, 9, 8592-8621 zu entnehmen. Die EP 2 165 997 A1 offenbart eine ODHE unter Einsatz eines auf einem Molekularsieb basierenden Katalysators, bei dem ebenfalls Kohlendioxid umgesetzt wird. Zielrichtung der weiteren Umsetzung von Ethylen und Kohlenmonoxid aus der ODHE ist sodann explizit die Herstellung von Alkylpropionaten oder -estern.

Beispielhafte einzelne Katalysatorsysteme sind in der hier nicht weiter genannten weiterführenden Literatur beschrieben. Dabei sind insbesondere zu Cr-basierten Systemen zahlreiche Arbeiten bekannt. Alle Veröffentlichungen beschränken sich jedoch auf das Katalysatorsystem für eine kohlendioxidbasierte ODHE, ohne dabei technisch umsetzbare Verfahrenskonzepte zu vorzuschlagen oder eine Integration mit weiteren Reaktionsschritten zu berücksichtigen. Lediglich S. Arnob et al., "Design Project Report for the Production of Polymer Grade Ethylene via the Oxidative Dehydrogenation of Ethane", J. Innov. Sci. Eng. Technol. 2020, 1, 13-28, beschreiben prinzipielle Verfahrensschritte für eine kohlendioxidbasierte ODHE, die jedoch ebenfalls mit der Bereitstellung von Polymerethylen endet und nur eine grundlegende Beschreibung von Verfahrensschritten beinhaltet.

Trotz der zuvor zitierten Umsätze, Selektivitäten und Ausbeuten hat das Entwicklungsstadium jedoch den Labormaßstab noch nicht überschritten. Die in der Literatur genannten Werte beziehen sich zumeist auf den Labormaßstab und sind auf industrielle relevant Bedingungen nicht übertragbar (siehe beispielsweise A.S. Al-Awadi et al., "Dehydrogenation of Ethane to Ethylene by CO2 over Highly Dispersed Cr on Large-Pore Mesoporous Silica Catalysts", Catalysts 2020, 10, 97. Gründe sind zunächst eine oft rasche Katalysatordeaktivierung bei der ausschließlichen Verwendung von Kohlendioxid als Oxidationsmittel und eine unter relevanten Bedingungen zumeist relativ niedrige Raumgeschwindigkeit, die ein hohes Katalysatorvolumen erfordert. Vor allem wird das entstehende Kohlenmonoxid als Koppelprodukt in bisherigen Ansätzen als unerwünschtes Nebenprodukt betrachtet und erfordert eine aufwendige Abtrennung aus dem Produktgemisch der ODHE.

Ebenso wurden z.B. in WO 20157113747 A1 Ansätze in Erwägung gezogen, um Kohlenmonoxid und Kohlendioxid nicht als Oxidationsmittel einzusetzen, sondern in der Hauptfunktion als Verdünnungsmittel. Dieses kann aus dem Zerlegungsteil einer entsprechenden Anlage recycliert werden. Es kann auch die Zuführung von mindestens stöchiometrischen Mengen an Sauerstoff erfolgen, wodurch eine Bildung von Kohlendioxid gefördert wird und Kohlendioxid eben nicht als Oxidationsmittel dient. Kohlenmonoxid wird lediglich als Nebenprodukt in geringen Mengen erwähnt und soll insbesondere, z.B. mittels katalytischer Oxidation, weiter in Kohlendioxid überführt werden.

Eine neuere Anmeldung (WO 2020/074750 A1) beschreibt neben der Verwendung von Kohlendioxid als Verdünnungsmittel auch die Verwendung von Kohlenmonoxid als Verdünnungsmittel. In dieser Anmeldung wird explizit die Verwendung von MoVNbOₓ- bzw. MoVNbTeOₓ-Katalysatoren beschrieben und beansprucht. Die in dieser Schrift erwähnte (zusätzliche) Verwendung von Dampf als Verdünnungsmittel ist aus zahlreichen anderen Schriften ebenfalls als Stand der Technik wohlbekannt. Für eine weitergehende Nutzung oder gar gezielte Herstellung nennenswerter Mengen an Kohlenmonoxid in der ODH(E) ist jedoch jedoch keine geeignete Lösung offenbart.

Eine weitere Anmeldung (US 10,647,635 B2) beschreibt ebenfalls die Einspeisung von Kohlendioxid in den Einsatz einer ODH(E). Als Katalysator wird ebenfalls MoVNbTeOₓ als Basismaterial verwendet, es kann jedoch noch zusätzlich ein Zusatz von Pd erfolgen. Insbesondere wird die Austrittskonzentration von Kohlendioxid als Funktion aus zugeführter Menge an Kohlendioxid und umgesetzter Menge an Kohlendioxid beschrieben. Entsprechend den Beispielen wird Kohlendioxid insbesondere umgesetzt, was sich dann in negativen Kohlendioxidselektivitäten niederschlägt. Allerdings wird auch hier keine gezielte Kohlenmonoxidproduktion angestrebt, und in den aufgeführten Beispielen liegt das Verhältnis von Ethylen zu Kohlenmonoxid durchgehend bei mehr als 10 zu 1, teilweise sogar mehr als 20 zu 1. Zusätzlich wird hier auch die bislang inhärente Eigenschaft der Essigsäurekoproduktion unter technisch relevanten Bedingungen adressiert und in den Beispielen quantifiziert. Laut weiteren technischen Ausführungen bewirkt eine Erhöhung des Dampfanteils im Reaktionseinsatz eine Erhöhung der Oxidationsaktivität des enthaltenen Kohlendioxid.

Beide zuletzt genannten Schriften führen Ausführungsbeispiele einer ODHE in einem Temperaturbereich von ca. 300 bis 350 °C auf, wie e r für die verwendeten MoVNbTeOₓ-Katalysatoren typisch ist.

Gemäß Stand der Technik erfolgt die oxidative Dehydrierung vorzugsweise in Festbettreaktoren, insbesondere in gekühlten Rohrbündelreaktoren, z.B. mit Salzschmelzekühlung. Für stark exotherme Reaktion, also insbesondere oxidative Reaktionen, wozu auch die ODH(E) zählt, ist dabei allgemein die Verwendung eines Reaktorbettes mit mehreren Zonen bekannt. Grundlagen hierzu sind beispielsweise in WO 2019/243480 A1 beschrieben. Diese Schrift beschreibt dabei das Prinzip, dass verschiedene Katalysatorbetten bzw. entsprechende Reaktionszonen, die unterschiedliche Katalysatorbeladungen und/oder Katalysatoraktivitäten pro Raumeinheit aufweisen, zum Einsatz kommen.

Die Führung des Kühlmediums - beispielsweise einer Salzschmelze - in einem Rohrbündelreaktor bei der ODH kann dabei im Gleich- oder Gegenstrom zum Reaktionseinsatz gewählt werden. Bei Führung des Kühlmediums - insbesondere einer Salzschmelze - im Gegenstrom lässt sich ein besonderer zusätzlicher Vorteil erzielen, da hier die Reaktionswärme aus der Hauptreaktion in der reaktiven Vorwärmzone teilweise mit ausgenutzt werden kann. Ebenso sind unterschiedliche Kühlkreisläufe in Kombination mit unterschiedlichen Katalysatorlagen denkbar (wie auch in WO 2019/243480 A1 aufgeführt).

Ferner sind ggf. der eigentlichen ODH(E) nachgeschaltete geeignete Reinigungsschritte und Entfernung von Spurenkomponenten, insbesondere Restgehalte von Sauerstoff aus der ODH(E) und gebildete Acetylenverbindungen zu berücksichtigen.

Zur Entfernung von Acetylen aus einem Prozessgas von Ethylenanlagen werden z.B. allgemein isotherme und adiabate Hydrierungen verwendet, wie hier nicht näher ausgeführt. Bei der etablierten Dampfspalt-Technologie finden all diese Hydrierungen in Abwesenheit von molekularem Sauerstoff und bei Kohlenmonoxidgehalten von deutlich weniger als 1% statt (typischer Bereich: max. 1000 ppm). Die Anwesenheit hoher Kohlenmonoxidgehalte erfordert daher zumeist andere Ansätze: Eine kombinierte Acetylen-Sauerstoff-Entfernung direkt am Austritt des ODHE-Reaktors ist dabei beispielsweise in der US 2010/0256432 A1 beschrieben. Eine gezielte Anordnung einer Acetylenhydriereinheit in der Trennsequenz einer ODHE-Anlage wurde ebenfalls in Erwägung gezogen. Zusätzlich kann dabei die Hydriereinheit derart betrieben werden, dass sie gleichzeitig als Sauerstoffentfernungseinheit funktioniert. Ein solcher Ansatz wird beispielsweise in WO 2020/187572 A1 beschrieben. Eine teilweise Entfernung des Sauerstoffs und des oder der Acetylene erfolgt dabei vorteilhafterweise, nachdem das Prozessgas der ODHE oder ein Teil hiervon teilweise oder vollständig einer Kondensatabscheidung und einer Verdichtung unterworfen wurde, und bevor ein nach der zumindest teilweisen Entfernung von Sauerstoff und Acetylen(en) verbleibendes Gasgemisch zumindest teilweise einer oder mehreren Stufen einer Kohlendioxidentfernung unterworfen wird.

Typischerweise muss im Rahmen einer geeigneten Verfahrensführung bei einem ODH(E)-Prozess sodann das im Produktstrom enthaltene Kohlenmonoxid entfernt werden. Dazu kommt üblicherweise eine Trennung zum Einsatz, die entsprechende kryogene Bedingungen erfordert. Schlussendlich muss nicht umgesetztes Ethan von Ethylen getrennt werden, was typischerweise mittels eines Splitters, der ebenfalls unter kryogenen Bedingungen betrieben wird, erfolgt.

Wie auch nachfolgend erläutert, koppelt die vorliegende Erfindung die ODH(E) mit einer Hydroformylierung, ggf. mit nachgeschalteter Hydrierung und Dehydratisierung. Zum Hintergrund wird an dieser Stelle beispielsweise auf Z. Xie et al., "Reactions of CO2 and ethane enable CO bond insertion for production of C3 oxygenates", Nat. Commun. 2020, 11, 1887, verwiesen, wo bereits die Kombination einer ausschließlich kohlendioxidbasierten ODHE mit einer Hydroformylierung beschrieben ist. Für die ODHE kommt dabei ein Fe₃Ni/CeO₂-Katalysator zum Einsatz, der oberhalb von 600 °C betrieben wird. Erst ab ca. 700 °C werden nennenswe rte Ethylenausbeuten erzielt. Wie bereits zuvor ausgeführt, ist in diesem Temperaturbereich bereits mit thermischer Dehydrierung zu rechnen und entsprechend enthält das Produktgemisch bereits nennenswerte Anteile von Wasserstoff. Der erhaltene Produktstrom wird sodann einem geträgerten Rh-Katalysator zugeführt und bei Temperaturen um 200 °C einer Hydroformylierung unterworfen. Die Veröffentlichung beschränkt sich dabei auf Laborergebnisse und offenbart insbesondere keine Lösung für eine integrierte technische Verfahrensführung.

Zusätzlich werden weiter unten die Reformierung, Trocken- bzw. Kohlendioxidreformierung (Dryreforming), partielle Oxidation (POX), Vergasung und Elektrolyse als bekannte Verfahren zur Bereitstellung von Wasserstoff bzw. Synthesegas genannt, wobei auch eine Wassergasshift zur gezielten Einstellung des Verhältnisses von Wasserstoff zu Kohlenmonoxid zum Einsatz kommen kann. Auch hier sind Grundlagen jeweils in den genannten Veröffentlichungen zu finden. Generell sollen hier unter einer "Wassergasshift" immer beide Richtungen der mit diesem Begriff bezeichneten Gleichgewichtsreaktion verstanden werden, also die Reaktion von Kohlenmonoxid mit Wasser zu Kohlendioxid und Wasserstoff und umgekehrt (letztere auch als "reverse" Wassergasshift, RWGS, bezeichnet). Die Richtung der Wassergasshift wird hier dem jeweiligen Bedarf entsprechend eingesetzt.

Eine Reihe unterschiedlicher Trenn- und Aufreinigungsschritte ist bekannt. Beispielsweise kann im Rahmen der vorliegenden Erfindung eine Kohlendioxidentfernung mittels Absorption (z.B. regenerierbare Aminwäschen und nicht regenerierbare Laugewäschen) zum Einsatz kommen. Das beladene Waschmittel bei Aminwäschen wird dann in einer separaten Kolonne regeneriert. Hierbei wird reines Kohlendioxid durch Desorption freigesetzt und steht als relativ reiner Strom für eine weitere Verwendung zur Verfügung. Gegebenenfalls können auch weitere absorptive Verfahren, Phasentrennungen, insbesondere mittels Abscheidern und Dekantern, adsorptive Reinigungs- und Trocknungsverfahren, die je nach Material und Verwendungszweck regenerierbare und nicht regenerierbare Adsorbentien verwenden, katalytische Spurenentfernungen, insbesondere zur Entfernung von Spuren von Acetylen(en) und Sauerstoff, kryogene und nicht-kryogene Destillationsverfahren, insbesondere eine Extraktivdestillation, eine Druckwechseladsorbtion (PSA) und Membranverfahren. zum Einsatz kommen.

### Merkmale und Vorteile der Erfindung

Grundsätzlich wird die eingangs genannte Aufgabenstellung durch eine geeignete Kopplung von Verfahrensschritten (Ethylenerzeugung, Hydroformylierung sowie ggf. Hydrierung und Dehydratisierung) gelöst. Zusätzlich können insbesondere auch geeignete Trenn- und Reinigungsschritte berücksichtigt werden.

Während bisherige Ansätze zur ODHE darauf abzielen, die Bildung von Nebenprodukten und insbesondere von Kohlenstoffoxiden möglichst weit zu minimieren, beruht die vorliegende Erfindung gerade auf dem gegenteiligen Ansatz und nutzt gebildetes Kohlenmonoxid als Baustein in der weiteren Wertschöpfungskette. Erfindungsgemäß wird also ein ausreichend hoher Anteil an Kohlenmonoxid im Produktstrom der ODHE angestrebt und die ODHE im Gegensatz zu bekannten Verfahren gerade so betrieben, dass signifikante Mengen an Kohlenmonoxid (bis 1 zu 1 im Verhältnis zu Ethylen) erzeugt werden. Der Anteil an Kohlenmonoxid im Produktstrom der ODHE beträgt dabei mindestens 5%, 10%, 25%, 50%, 75% und bis zu 100% Kohlenmonoxid im Verhältnis zu Ethylen.

Es wird insgesamt ein Verfahren zur Herstellung einer Zielverbindung vorgeschlagen, bei dem ein erstes Komponentengemisch unter Erhalt eines zweiten Komponentengemischs einer oxidativen Dehydrierung unterworfen wird, und bei dem zumindest ein Teil des zweiten Komponentengemischs unter Erhalt eines dritten Komponentengemischs einer Hydroformylierung unterworfen wird, wobei das erste Komponentengemisch ein Paraffin (im Falle der ODHE Ethan), das zweite Komponentengemisch ein in der oxidativen Dehydrierung aus dem Paraffin gebildetes Olefin (im Falle der ODHE Ethylen) und das dritte Komponentengemisch eine in der Hydroformylierung aus dem Olefin gebildete Folgeverbindung (im betrachteten Fall insbesondere Propanal), die die Zielverbindung oder eine zu der Zielverbindung umsetzbare Vorläuferverbindung darstellt, enthalten. Bei der Zielverbindung kann es sich insbesondere um Propylen handeln, das aus dem Propanal über 1-Propanol durch eine an sich bekannte Hydrierung und Dehydratisierung gebildet werden kann.

Das erste Komponentengemisch weist erfindungsgemäß Sauerstoff in einem Sauerstoffgehalt und/oder Kohlendioxid in einem Kohlendioxidgehalt auf, und das zweite Komponentengemisch weist Kohlenmonoxid in einem Kohlenmonoxidgehalt und das Olefin in einem Olefingehalt auf, wobei der Kohlenmonoxidgehalt in dem zweiten Komponentengemisch erfindungsgemäß bei 5% bis 120% des Olefingehalts liegt. Der Kohlenmonoxidgehalt kann insbesondere bei mehr als 10%, 25%, 50% oder 75% und bis zu 110% des Olefingehalts liegen oder in einem der vorstehend genannten Wertebereiche.

Die vorliegende Erfindung deckt den eingangs erwähnten zunehmenden Propylenbedarf (insbesondere auch im Verhältnis zu Ethylen) deutlich besser als bekannte Dampfspaltverfahren, ggf. aber auch einen vorhandenen Bedarf an den Intermediaten Propanal und 1-Propanol, je nachdem, welche dieser Verbindungen als Zielverbindung betrachtet wird. Die Erfindung ermöglicht es, auf aufwendige Reinigungsschritte zur Abtrennung von Kohlenstoffoxiden zu verzichten und diese in einem integrierten Gesamtprozess zu verwerten. Die Erfindung ermöglicht eine deutliche Reduzierung des Kohlendioxid-Footprints (petro-)chemischer Anlagen und erlaubt auch eine zusätzliche stoffliche Verwertung von Kohlendioxid auch aus anderen Quellen wie Verbrennungsprozessen.

Insgesamt schafft die Erfindung also ein besonders effizientes Verfahren zur Propylenherstellung aus Ethan, die stoffliche Verwertung von Kohlendioxid und entsprechende Minimierung des Kohlendioxid-Footprints und die vorteilhafte Nutzung der als Nebenprodukte in der ODHE gebildeten Kohlenstoffoxide. Neben einer Minimierung des Aufwandes in der Produktaufreinigung und -zerlegung, insbesondere der Vermeidung kryogener Trennungen in der Trennung ggf. Trocknung ergibt sich eine deutliche Verbesserung der Energieeffizienz.

Die Nachteile bestehender Technologien wie Steamcracking sowie MTO und MTP, die Ethylen und Propylen immer nur in einem bestimmten Verhältnis produzieren, welches sich in Abhängigkeit vom Einsatz durch Anpassung der Prozessparameter (z.B. Temperatur, Verweilzeit etc.) nur bedingt einstellen lässt, werden durch die vorliegende Erfindung überwunden.

Die Erfindung schafft auch Vorteile beispielsweise gegenüber der bekannten Olefinmetathese, die zwar eine flexible Umwandlung von Ethylen in Propylen ermöglicht, heutzutage aber von den Olefinen Ethylen und 2-Buten ausgeht, die zu Propylen umgesetzt werden. Es handelt sich prinzipiell um eine Gleichgewichtsreaktion und ursprünglich wurde diese Technologie für die umgekehrte Umsetzung, also von Propylen zu Ethylen und Buten entwickelt. Das notwendige 2-Buten kann z.B. aus der selektiven Dimerisierung von Ethylen oder als Fraktion aus der Produktion von linearen α-Olefinen ebenfalls basierend auf der Oligomerisierung von Ethylen gewonnen werden. Ebenfalls kann 1-Buten eingesetzt werden, welches isomerisiert werden kann. Ebenso ist auch die Verwendung von höheren internen linearen Olefinen bekannt, wobei sich durch entsprechende Reaktionskaskaden letztlich schlussendlich immer Propylen als Zielprodukt ergibt. Auch wenn die Olefinmetathese-Technologie als ausgreift und etabliert angesehen werden kann, bleibt als ein wesentlicher Nachteil, dass im Gegensatz zur vorliegenden Erfindung bereits hochwertige und hochpreisige Edukte verwendet werden. Die Olefinmetathese dient daher zumeist insbesondere dazu, eine flexible Ausbilanzierung von Ethylen- und Propylenproduktion und -bedarf an einem spezifischen Standort oder Verbund zu ermöglichen.

Ein Nachteil bekannter Verfahren, den die vorliegende Erfindung ebenfalls überwindet, ist insbesondere auch die Bildung signifikanter Mengen an Kohlendioxid, entweder aus einer Befeuerung, da es sich um endotherme Prozesse handelt (Dampfspalten und PDH) bzw. in Form von Nebenprodukten (klassische ODHE). Kohlendioxid im Prozessgas der genannten Verfahren (einschließlich der ODHE) kann zwar durch entsprechende regenerative Wäschen abgetrennt und als Reinfraktion erhalten werden, bislang erfolgt jedoch keine weitere integrierte Verwertung insbesondere von Kohlendioxid innerhalb der entsprechenden Verfahren. Die Erfindung vermeidet, wie erwähnt, eine aufwendige Abtrennung von Nebenprodukten, insbesondere durch Verwendung kryogener Destillationen zur Abtrennung von beispielsweise Wasserstoff, Methan und Kohlenmonoxid sowie weiterer Kohlenwasserstoffe beim Dampfspalten und von Kohlenmonoxid bei der klassischen ODHE.

Das erste Komponentengemisch kann in einer Ausgestaltung der vorliegenden Erfindung weniger als 25%, weniger als 10% oder weniger als 5% Kohlenmonoxid, 0,1 bis 50%, 5 bis 40% oder 10 bis 25% Kohlendioxid, 20 bis 85% oder 30 bis 60% Ethan, weniger als 20%, weniger als 10%, weniger als 5% oder weniger als 1% Ethylen, und 0,1 bis 30%, 2 bis 25% oder 5 bis 20% Sauerstoff, jeweils auf Volumenbasis und ohne Berücksichtigung des durch Wasser gebildeten Anteils, enthalten.

Das zweite Komponentengemisch kann im Rahmen der vorliegenden Erfindung insbesondere 1% bis 60% oder 5% bis 40% Kohlenmonoxid, 0,1% bis 40%, 0,5% bis 25% oder 5 bis 20% Kohlendioxid, 0,1% bis 50%, 5% bis 40% oder 10% bis 30% Ethan, 10% bis 80% oder 30% bis 70% Ethylen und weniger als 10%, weniger als 5%, weniger als 1% oder weniger als 0,1% Sauerstoff, jeweils auf Volumenbasis und ohne Berücksichtigung des durch Wasser gebildeten Anteils, enthalten.

Die obigen Angaben beziehen sich dabei, ausgedrückt durch "ohne Berücksichtigung des durch Wasser gebildeten Anteils", jeweils auf den trockenen Anteil entsprechender Gemische, die je nach Verfahrensführung also zusätzlich noch Wasserdampf aufweisen können. Insbesondere kann das erste Komponentengemisch 1% bis 95%, 5% bis 50% oder 10% bis 35% Wasser, auf Volumenbasis und bezogen auf das gesamte erste Komponentengemisch, enthalten.

Weitere Komponenten wie Oxygenate, also Aldehyde, Ketone, Ether etc., können in Spuren, d.h. typischerweise zu weniger als 0,5 Molprozent, insbesondere zu weniger als 0,1 Molprozent, in Summe in entsprechenden Gemischen enthalten sein.

Der erfindungsgemäß erhöhte Kohlenmonoxidanteil im Produktstrom der ODHE kann dabei insbesondere durch eine Einspeisung und ggf. auch Rückführung von Kohlendoxid in die ODHE bzw. einen entsprechenden ODHE-Reaktor erreicht werden. Für die Verwertung von Kohlenstoffoxiden kommen im Rahmen der vorliegenden Erfindung also folgende Ansätze zum Tragen:
1. In der ODHE entstehendes Kohlenmonoxid wird als Synthesebaustein in einer nachgeschalteten Hydroformylierung von Ethylen zu Propanal als Wertprodukt und ggf. Synthesebaustein genutzt.
2. Kohlendioxid wird in die ODHE eingespeist, um insbesondere den Anteil an Kohlenmonoxid im Produktstrom der ODHE zielgerichtet zu erhöhen. Hier erfolgt also entsprechend Punkt 1 dann eine stoffliche Verwertung von Kohlendioxid und der Prozess wirkt als Kohlendioxidverbraucher bzw. Kohlendioxidsenke.
3. Kohlendioxid im Produktstrom der ODHE kann wiederum in die ODHE rezykliert werden. Somit können insbesondere Kohlendioxidemissionen aus der ODHE effizient vermieden werden.

Insgesamt kann also in Summe ein Kohlendioxidverbrauch erzielt werden, was im Ergebnis als "negative" Kohlendioxidemission betrachtet werden kann.

Bisher werden Kohlenstoffoxide, insbesondere Kohlenmonoxid, als Nebenkomponenten in untergeordneten Mengen (insbesondere im Verhältnis zu Ethylen) im Produktstrom der ODHE betrachtet. Es erfolgt hier also keine weitere Optimierung (insbesondere keine Erhöhung) des Kohlenmonoxidanteils im Produktstrom der ODHE.

Wie erwähnt, kann in der oxidativen Dehydrierung als weiteres Nebenprodukt insbesondere eine Carbonsäure, im Falle von Ethan als Einsatz in die oxidative Dehydrierung insbesondere Essigsäure, gebildet werden. Diese Essigsäure kann, zusammen mit Reaktionswasser, vergleichsweise einfach durch eine Kondensation und/oder eine Wasserwäsche aus einem entsprechenden Produktgemisch der oxidativen Dehydrierung abgetrennt werden. Durch geeignete, dem Fachmann bekannte Verfahren ist bedarfsweise dann auch eine Anreicherung der Essigsäure und Gewinnung als eigenes Wertprodukt möglich.

Im Rahmen der vorliegenden Erfindung wird sodann zumindest ein Teil des Produktstromes der ODHE (ausgedrückt mit "zumindest ein Teil des zweiten Komponentengemischs") einer Hydroformylierung unter Erhalt von Propanal umgesetzt. Dieses Propanal kann als die Zielverbindung fungieren oder weiteren Schritten, insbesondere einer Hydrierung und ggf. nachgeschalteten Dehydratisierung unterworfen werden, in denen dann jeweils die Zielverbindung erhalten wird. Mit anderen Worten kann zumindest ein Teil des in der Hydroformylierung in einer Ausgestaltung gebildeten Propanals einer weiteren Umsetzung zu 1-Propanol unterworfen werden.

Die Folgeschritte Hydroformylierung und/oder Hydrierung benötigen des Weiteren Wasserstoff und ggf. auch eine Anpassung (insbesondere Erhöhung) der Menge an Kohlenmonoxid. Es ist also die Bereitstellung von Wasserstoff für die Hydroformylierung und ggf. für weitere Folgeschritte wie die Hydrierung sowie bedarfsweise auch die Einstellung des benötigten/gewünschten Kohlenmonoxidanteils aus geeigneter Quelle erforderlich. Dies können z.B. Verfahren wie Dryreforming oder Partialoxidation sein, Synthesegase können aber auch durch eine Vergasung von diversen Einsätzen (Kohle, Öl, Abfälle, insbesondere Kunststoffabfälle etc.) erzeugt werden. Alternativ kommen aber auch existierenden Ströme in petrochemischen Komplexen als Wasserstoff- und/oder Kohlenmonoxidquelle, beispielsweise aus einer sogenannten Demethanisierung, in Betracht.

Mit anderen Worten kann die Erfindung also vorsehen, unter Verwendung des zweiten Komponentengemischs einen Reaktionseinsatz zu bilden, der durch Zugabe von Wasserstoff und/oder Kohlenmonoxid gegenüber dem zweiten Komponentengemisch an Wasserstoff und/oder Kohlenmonoxid angereichert ist, und welcher sodann der Hydroformylierung unterworfen wird.

Vorzugsweise wird im Rahmen der vorliegenden Erfindung dabei Wasserstoff aus einer Reformierung von Kohlenwasserstoffen, insbesondere von Methan, verwendet, wobei sich der Anteil an Kohlenmonoxid im entsprechenden Synthesegasstrom durch eine Wassergasshift (in beliebiger Richtung, siehe oben) bedarfsgerecht einstellen lässt. Auch in dem Reformingschritt bzw. in der Wassergasshift entstehendes Kohlendioxid kann durch geeignete Prozessführung wiederum zumindest teilweise der ODHE zugeführt und stofflich verwertet werden. In einer bevorzugten Ausführung der Erfindung kann auch eine Wassergasshift auch stromauf der Hydroformylierung vorgesehen sein. Auf diese Weise kann unter Bereitstellung entsprechender Wasserstoffmengen auch im Austrittsstrom der ODHE enthaltenes Kohlendioxid in Kohlenmonoxid überführt werden. Ein besonderer Vorteil der Erfindung ergibt sich an dieser Stelle also auch daraus, dass der Wasserstoff keine besonders hohe Reinheitsanforderungen erfüllen muss, sondern Anteile von Kohlenmonoxid und/oder insbesondere Kohlendioxid nicht nur toleriert, sondern diese im Rahmen des Gesamtprozesses sogar stofflich verwertet werden können. Grundsätzlich ist aber auch eine mindestens teilweise Einspeisung von Wasserstoff aus einer Elektrolyse möglich.

Zusammengefasst kann in einer Ausgestaltung der Erfindung vorgesehen sein, dass stromauf der Hydroformylierung Wasserstoff und/oder Kohlenmonoxid eingespeist und ein Verhältnis zwischen Wasserstoff und Kohlenmonoxid in einer Wassergasshift-Reaktion eingestellt wird.

Der Produktstrom der ODHE beinhaltet neben Ethylen weitere Komponenten wie insbesondere Ethan, Kohlendioxid und Kohlenmonoxid, ggf. können auch Spuren von Methan, Acetylen und/oder höheren Kohlenwasserstoffen enthalten sein. Ebenso können wie zuvor ausgeführt auch Oxygenate enthalten sein. Hinzu kommt die zuvor beschriebene Einspeisung von Wasserstoff und/oder Kohlenmonoxid in den Prozess. Ethan kann auch als Nebenprodukt in der Hydroformylierung gebildet werden.

Diese Komponenten gilt es in geeigneter Weise in einer vorteilhaften Ausgestaltung des erfindungsgemäßen Prozesses abzutrennen und möglichst an eine geeignete Stelle zurückzuführen und zu verwerten. Kohlendioxid kann aufgrund seiner hohen Wechselwirkung mit geeigneten Lösungsmitteln bzw. Waschflüssigkeiten vergleichsweise einfach aus dem Produktgemisch entfernt werden, wobei bekannte Verfahren zur Kohlendioxidentfernung, insbesondere entsprechende Wäschen (beispielsweise Aminwäschen) zum Einsatz kommen können. Eine weitere Feinreinigung und Reduzierung des Kohlendioxidgehaltes ist durch eine Laugewäsche möglich. Kohlendioxid kann daher also bedarfsweise abgetrennt und in der zugehörigen Regenerierung nahezu als Reinsubstanz gewonnen werden. Eine solche Kohlendioxidentfernung kann an geeigneter Stelle des Prozesses, insbesondere stromauf der Hydroformylierung erfolgen. Es ist aber auch eine Kohlendioxidentfernung an anderer Stelle, wie z.B. stromab des Kopfstromes einer Trennung von Kohlenwasserstoffen mit zwei und drei Kohlenstoffatomen, wie weiter unten beschrieben, möglich. Ein besonderer Vorteil der Positionierung der Kohlendioxidentfernung stromauf der Hydroformylierung ergibt sich daraus, dass einerseits keine Beeinflussung der Hydroformylierung durch Kohlendioxid erfolgt und andererseits die Kohlendioxidentfernung nicht durch Spuren von Propanal beeinträchtigt wird, die ggf. mit einem Amin reagieren können oder aber in einer Laugewäsche durch Aldolkondensation störende Polymerisate bilden können.

Das auf diese Weise erhaltene Kohlendioxid wird vorzugsweise zumindest teilweise wieder der ODHE als Reaktionseinsatz zugeführt, kann aber zumindest anteilig auch anderen Verfahren, wie z.B. Dryreforming, zugeführt werden.

Die oxidative Dehydrierung wird im Rahmen der vorliegenden Erfindung vorteilhafterweise auf einem Druckniveau von 1 bis 10 bar, insbesondere 2 bis 6 bar, und die Hydroformylierung sowie die Entfernung von Kohlendioxid werden vorteilhafterweise auf einem Druckniveau von 15 bis 100 bar, insbesondere 20 bis 50 bar, durchgeführt.

In einer besonders bevorzugten Ausgestaltung der vorliegenden Erfindung wird daher ein Produktgemisch aus der oxidativen Dehydrierung, hier als zweites Komponentengemisch bezeichnet, insbesondere nach einer Kondensatabtrennung, auf ein Druckniveau verdichtet, auf dem sowohl das Kohlendioxid aus der oxidativen Dehydrierung vor und/oder nach der Hydroformylierung abgetrennt als auch die Hydroformylierung durchgeführt werden. Zwischen der Abtrennung von Kohlendioxid und der Hydroformylierung stromauf und/oder stromab hiervon können dabei ggf. auch zusätzliche Zwischenschritte vorgesehen sein. Beide Verfahren erfolgen im Wesentlichen auf dem gleichen Druckniveau, was insbesondere bedeutet, dass zwischen den beiden keine zusätzliche Verdichtung erfolgt und sich der genaue Betriebsdruck beider Schritte nur durch die prozessbedingten Druckverluste zwischen beiden Schritten ergibt.

Dabei stellt das Druckniveau, auf dem die Entfernung von Kohlendioxid und die Hydroformylierung betrieben werden, bevorzugt das höchste Druckniveau im Gesamtprozess dar.

Im Rahmen der vorliegenden Erfindung können sodann an geeigneter Stelle, insbesondere stromab der Hydroformylierung und/oder stromab einer Hydrierung, grundsätzlich bekannte Einrichtungen zur Abtrennung zumindest eines Teils der leichten Komponenten eingesetzt werden. Unter leichten Komponenten sollen hier insbesondere Wasserstoff, Kohlenmonoxid, Methan, Ethan und/oder Ethylen sowie entsprechende Gemische dieser Verbindungen verstanden werden. In Abhängigkeit der Position einer Kohlendioxidentfernung kann überdies auch Kohlendioxid enthalten sein. Prinzipiell können diese leichten Komponenten die Hydroformylierung als inerte Verbindungen durchlaufen (z.B. Ethan, Kohlendioxid und/oder Methan), die lediglich den Partialdruck der Reaktanden beeinträchtigen oder es handelt sich um die Reaktanden der Hydroformylierung (Ethylen, Kohlenmonoxid, Wasserstoff).

Die Einrichtungen zur Abtrennung von leichten Komponenten können insbesondere eine adsorptive Trennung, eine absorptive Trennung, eine Membrantrennung, eine destillative Trennung und/oder eine Phasentrennung umfassen. Unter einer "Phasentrennung" soll dabei eine reine Trennung von flüssiger Phase und Gasphase nach Abkühlung in einem keine typischen Trenneinrichtungen einer Rektifikationskolonne aufweisenden Trennbehälter verstanden werden.

Typischerweise kommt beispielsweise stromab der Hydroformylierung eine Trennung von Kohlenwasserstoffen mit zwei und drei Kohlenstoffatomen zum Einsatz. Dies kann insbesondere eine Extraktivdestillation sein, bei der insbesondere Propanol, welches in einer nachgeschalteten Hydrierung von Propanal gebildet wird, als ein Extraktionsmittel verwendet werden kann. Auf diese Weise können insbesondere kryogene Bedingungen vermieden werden. Aufgrund des minimierten apparativen Aufwandes kann alternativ auch ein reiner Abscheider, bei dem nach Abkühlung auf moderate (nicht-kryogene) Temperaturen das Produkt der Hydroformylierung (Propanal) als Flüssigphase anfällt, besonders vorteilhaft sein.

Allgemein sollen hier unter "nicht-kryogenen" Bedingungen solche Bedingungen verstanden werden, die ein Temperaturniveau von nicht weniger als -20 °C, insbesondere nicht weniger als 0 °C, insbesondere nicht weniger als Kühlwassertemperatur von 5 bis 25 °C, umfassen. Bei einer reinen Phasentrennung in der Flüssigphase gelöste leichtere Moleküle sind dabei nicht störend, da diese durch die eine weitere Integration wie hier vorgeschlagen abgetrennt und einer weiteren stofflichen Verwertung zugeführt werden können. Die Abtrennung der soeben genannten Nebenprodukte erfolgt dabei in vorteilhafter Weise vollständig nichtkryogen und gestaltet sich daher apparativ und hinsichtlich des Energieaufwands ausgesprochen einfach. Dies stellt einen wesentlichen Vorteil der vorliegenden Erfindung gegenüber Verfahren gemäß dem Stand der Technik dar, die typischerweise eine aufwendige Abtrennung von in nachfolgenden Verfahrensschritten unerwünschten Komponenten erfordern. Insbesondere müssen nicht vorher zwingend Kohlendioxid und Wasser (bei Temperaturen in den folgenden Prozessschritten über 0 °C) abgetrennt werden müssen, die zu Verlegungen bzw. Vereisungen in kryogenen Anlagenteilen führen würden.

Mit anderen Worten ist gemäß einer besonders vorteilhaften Ausgestaltung der Erfindung vorgesehen, dass das Verfahren stromab der oxidativen Dehydrierung und stromab der Hydroformylierung vollständig nicht-kryogen durchgeführt wird.

Erforderlichenfalls kann das erfindungsgemäße Verfahren auch notwendige Reinigungsschritte zur Entfernung von Spuren, insbesondere von Acetylen und Sauerstoff, an geeigneter Stelle enthalten. Diese sind vorteilhafterweise stromauf der Hydroformylierung positioniert, um eine mögliche Beeinträchtigung der Hydroformylierung zu vermeiden.

Beliebige im Rahmen der vorliegenden Erfindung anfallende, wasserhaltige Gasgemische können an jeweils geeigneter Stelle einer Trocknung unterworfen werden. Beispielsweise kann eine Trocknung stromab der Hydroformylierung erfolgen, wenn diese in einer Ausgestaltung der vorliegenden Erfindung in der wässrigen Phase erfolgt und die Hydrierung stromab der Hydroformylierung einen trockenen Strom als Reaktionseinsatz benötigt. Die Trocknung muss, falls dies für die nachfolgenden Verfahrensschritte nicht erforderlich ist, nicht bis zur vollständigen Trockne erfolgen, sondern es können ggf. auch Wassergehalte in entsprechenden Gas- bzw. Stoffgemischen verbleiben, soweit diese tolerabel sind. Auch unterschiedliche Trocknungsschritte können an unterschiedlichen Stellen des Verfahrens und ggf. mit unterschiedlichen Trocknungsgraden vorgesehen werden.

Da eine Hydroformylierung unter technisch relevanten Bedingungen üblicherweise nur 85 bis 95% Umsatz ergibt, wird insbesondere ein Teilstrom des Kopfproduktes der Trennung von Kohlenwasserstoffen mit zwei und drei Kohlenstoffatomen, die bevorzugt als Extraktivdestillation ausgeführt ist, in den Eintrittsstrom der Hydroformylierung zurückgeführt.

Für eine weitere Auftrennung der leichten Komponenten kommen des Weiteren eine Trennung von Kohlenwasserstoffen mit zwei Kohlenstoffatomen von leichteren Verbindungen, ein entsprechender Splitter sowie ggf. eine Wasserstoffabtrennung bzw. Wasserstoffrückgewinnung, z.B. durch eine PSA oder durch Membranverfahren in Betracht. Mit diesen Prozesschritten können bei Bedarf entsprechende Teilströme gebildet und an vorteilhafter Stelle in den Gesamtprozess zurückgeführt werden. Es ergeben sich dabei folgende Möglichkeiten: (i) Kohlendioxid kann in die ODHE zurückgeführt werden, (ii) Ethan kann in die ODHE zurückgeführt werden, (iii) Ethylen, Wasserstoff und Kohlenmonoxid können in die Hydroformylierung zurückgeführt werden, und/oder (iv) Wasserstoff kann darüber hinaus einer nachgeschalteten Hydrierung zugeführt werden.

Mit anderen Worten schlägt die vorliegende Erfindung neben der Rückführung von Kohlendioxid in die ODHE in vorteilhaften Ausgestaltungen insbesondere vor, dass unter Verwendung zumindest eines Teils des zweiten und/oder dritten Komponentengemischs eine Ethan enthaltende Fraktion gebildet und in die oxidative Dehydrierung (10) zurückgeführt wird, und/oder dass unter Verwendung zumindest eines Teils des dritten Komponentengemischs eine Ethylen, Wasserstoff und/oder Kohlenmonoxid enthaltende Fraktion gebildet und in die Hydroformylierung (20) zurückgeführt wird, und/oder dass unter Verwendung zumindest eines Teils des dritten Komponentengemischs eine überwiegend oder ausschließlich Wasserstoff enthaltende Fraktion gebildet und in einer Hydrierung verwendet wird.

Wird Kohlendioxid aus dem zweiten Komponentengemisch abgetrennt, wird vorteilhafterweise zumindest ein Teil des zweiten Komponentengemischs aus der oxidativen Dehydrierung auf ein Druckniveau verdichtet, auf dem das Kohlendioxid aus dem zweiten Komponentengemisch abgetrennt und die Hydroformylierung durchgeführt wird.

Um die Akkumulation von Spuren und Inerten zu vermeiden, kann bei Bedarf ein Teil des Kopfstromes der Trennung von Kohlenwasserstoffen mit zwei und drei Kohlenstoffatomen und/oder eines oder mehrerer der Rückführströme als Purgestrom aus dem Prozess herausgeführt werden. Unter (möglicherweise hypothetischen) idealen Bedingungen könnte ein entsprechender Purgestrom auch theoretisch ganz entfallen. Bei einem geringen Eintrag und/oder Bildung der genannten Nebenprodukte kann der Purgestrom entsprechend klein dimensioniert werden.

Wie bereits zuvor ausgeführt, können weitere geeignete Reaktions- und/oder Verfahrensschritte an geeigneter Stelle innerhalb des Prozesses angeordnet sein, um Nebenprodukte oder Spurenverbindungen zu entfernen.

Im Falle einer Hydrierung des Propanals zu Propanol kann an beliebiger geeigneter Stelle in dem erfindungsgemäßen Verfahren und seinen Ausgestaltungen eine Einspeisung von Wasserstoff erfolgen, insbesondere stromauf der optional vorgesehenen Hydrierung. Wasserstoff steht auf diese Weise für diese Hydrierung zur Verfügung. Die Einspeisung braucht nicht unmittelbar stromauf der Hydrierung zu erfolgen; vielmehr kann Wasserstoff auch durch stromauf der Hydrierung vorliegende bzw. durchgeführte Verfahrens- bzw. Trennschritte zugespeist werden.

Gemäß einer weiteren Ausgestaltung der vorliegenden Erfindung erfolgt bei der Umsetzung des Propanals zur eigentlichen Zielverbindung des erfindungsgemäßen Verfahrens eine Dehydratisierung des durch die Hydrierung gebildeten Propanols zu Propylen. Bei dieser Dehydratisierung des in der Hydrierung gebildeten Propanols bildet sich Wasser. Dieses fällt in einem Produktstrom an, der das Propylen und die Nebenkomponenten enthält. Dieser Produktstrom kann im Rahmen der vorliegenden Erfindung unter Erhalt eines das Propylen und die Nebenkomponenten enthaltenden und an Wasser abgereicherten Teilstroms ("Rohpropylenstrom") einer Wasserabtrennung unterworfen werden.

Dieser erhaltene Rohpropylenstrom hat typischerweise einen Propylengehalt von mindestens 80%, 90%, 95% oder 98%; mit anderen Worten kann in einer entsprechenden Ausgestaltung der Erfindung also das Propylen in einer Reinheit von mindestens 80%, 90%, 95% oder 98% erhalten werden.

In einer Ausgestaltung der Erfindung kann das Propylen in einem entsprechenden Rohpropylenstrom ganz oder teilweise unter Erhalt einer Reinpropylenfraktion einer oder mehreren geeigneten Fraktionierungen bzw. Fraktionierungsschritten einer oder mehrerer Fraktionierungen unterworfen werden.

Eine gebildete Wasserfraktion kann bei Bedarf z.B. zur Erzeugung von Prozessdampf oder auf andere Weise genutzt werden.

Insbesondere das bei einer Hydrierung des Propanals gebildete Propanol kann vergleichsweise einfach von nicht umgesetztem Ethan abgetrennt werden. Auf diese Weise kann also alternativ auch hier nichtkryogen ein Rückführstrom des Ethans und ggf. weiterer leichter Komponenten gebildet und wie zuvor beschrieben beispielsweise in die oxidative Dehydrierung zurückgeführt werden.

In dem erfindungsgemäß vorgeschlagenen Verfahren durchläuft vorteilhafterweise zumindest ein Teil des in der oxidativen Dehydrierung nicht umgesetzten Ethans die Hydroformylierung, wird stromab der Hydroformylierung abgetrennt, und wird zumindest teilweise in die oxidative Dehydrierung zurückgeführt.

Wie bereits zuvor mit anderen Worten ausgedrückt, umfasst eine vorteilhafte Ausgestaltung des Verfahrens, dass in der ODHE nicht umgesetztes und/oder entstehendes Kohlendioxid an geeigneter Stelle abgetrennt wird, wobei das Kohlendioxid zumindest teilweise in die ODHE zurückgeführt werden kann. In einer ebenfalls angesprochenen Ausgestaltung wird das Produktgemisch aus der oxidativen Dehydrierung, d.h. das zweite Komponentengemisch oder ein Teil hiervon, auf ein Druckniveau verdichtet, auf dem Kohlendioxid aus dem Produktgemisch der ODHE an geeigneter Stelle abgetrennt und die Hydroformylierung durchgeführt wird.

Stromauf der Hydroformylierung (und ggf. stromauf der Folgeschritte) werden insbesondere Wasserstoff und/oder Kohlenmonoxid zugeführt, und das Verhältnis zwischen Wasserstoff und Kohlenmonoxid in einem entsprechenden Strom, zuvor auch als Reaktionseinsatz bezeichnet, kann durch eine Wassergasshift-Reaktion eingestellt werden. Das Verfahren wird, wie erwähnt, stromab der oxidativen Dehydrierung und stromab der Hydroformylierung insbesondere im oben erläuterten Sinn vollständig nicht-kryogen durchgeführt.

Zu der erfindungsgemäß bereitgestellten Anlage und ihren Merkmalen sowie Ausgestaltungen hiervon sei auf die obigen Erläuterungen bezüglich des erfindungsgemäßen Verfahrens und seiner Ausgestaltungen ausdrücklich verwiesen, da diese eine entsprechende Anlage in gleicher Weise betreffen. Entsprechendes gilt insbesondere für eine Ausgestaltung einer entsprechenden Anlage, die vorteilhafterweise zur Ausführung eines entsprechenden Verfahrens in einer beliebigen Ausgestaltung eingerichtet ist.

Die Erfindung wird nachfolgend unter Bezugnahme auf die Figuren die Ausgestaltungen der Erfindung veranschaulichen, weiter erläutert.

### Kurze Beschreibung der Zeichnungen

Figur 1 veranschaulicht einen Prozess gemäß einer bevorzugten Ausführungsform der Erfindung in Form eines schematischen Ablaufplans. Figur 1 veranschaulicht dabei insbesondere die Grundverknüpfung der Prozesschritte.
Figur 2 veranschaulicht einen Prozess gemäß einer besonders bevorzugten Ausführungsform der Erfindung in Form eines schematischen Ablaufplans. Figur 2 beinhaltet insbesondere eine Verfahrensführung ohne kryogene Schritte.
Figur 3 veranschaulicht einen Prozess gemäß einer weiteren besonders bevorzugten Ausführungsform der Erfindung in Form eines schematischen Ablaufplans. Figur 3 beinhaltet insbesondere eine gezielte Auftrennung und Rückführung der leichten Komponenten unter Verwendung kryogener Schritte.
Figur 4 veranschaulicht einen Prozess gemäß einer weiteren besonders bevorzugten Ausführungsform der Erfindung in Form eines schematischen Ablaufplans. Figur 4 stellt eine besonders bevorzugte Auftrennung in den Recycleströmen dar.
Figur 5 veranschaulicht einen Prozess gemäß einer weiteren besonders bevorzugten Ausführungsform der Erfindung in Form eines schematischen Ablaufplans. Figur 5 beinhaltet insbesondere eine Verfahrensführung mit einer Wassergasshift-Reaktion vor der Hydroformylierung und ohne kryogene Schritte.

### Ausführliche Beschreibung der Zeichnungen

Ist nachfolgend von Prozess- oder Verfahrensschritten die Rede, sollen hierunter auch die jeweils für diese Verfahrensschritte verwendeten Apparate (insbesondere z.B. Reaktoren, Kolonnen, Wascheinrichtungen usw.) verstanden werden, auch wenn hierauf nicht ausdrücklich Bezug genommen wird. Allgemein gelten die einen Prozess betreffenden Erläuterungen für eine entsprechende Anlage jeweils in gleicher Weise. Die Begriffe "Prozess" und "Verfahren" werden synonym verwendet.

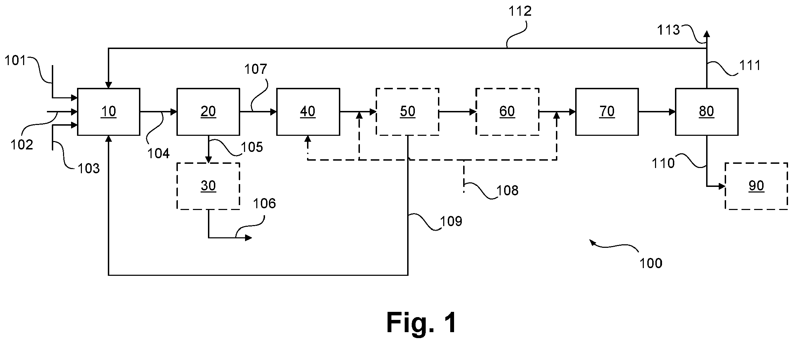

In der Figur 1 ist ein Prozess gemäß einer bevorzugten Ausgestaltung der vorliegenden Erfindung in Form eines schematischen Ablaufplanes veranschaulicht und insgesamt mit 100 bezeichnet.

In dem Prozess 100 werden einer ODHE 10 bzw. einem entsprechenden Reaktor die benötigten Reaktanden Ethan 101 und Sauerstoff 102 zugeführt. Ein entsprechender Strom von Ethan 101 kann dabei in Abhängigkeit von der Ethanquelle und evtl. vorgeschalteten Reinigungsschritten auch gewisse Anteile z.B. von Methan und/oder höheren Kohlenwasserstoffen enthalten. Zusätzlich können bedarfsweise Wasserdampf als Verdünnungsmittel (nicht explizit dargestellt) sowie Kohlendioxid 103 aus einer externen Quelle eingespeist werden. Ebenso können aber auch geeignete ethan- und kohlendioxidhaltige Gasgemische zum Einsatz kommen. Sämtliche der ODHE 10 zugeführten Komponenten bilden ein Einsatzgemisch, das hier auch als "erstes Komponentengemisch" bezeichnet wird.

Nach einer Abkühlung und Kondensation eines in der ODHE 10 erhaltenen Produktgemischs 104, hier als "zweites Komponentengemisch" bezeichnet (nicht dargestellt) erfolgt eine Phasentrennung 20 bzw. Kondensatabtrennung. Die dabei gebildete wässrige Phase 105, die Essigsäure enthalten kann, kann einer optionalen Essigsäureaufreinigung 30 unter Erhalt von Essigsäure 106 zugeführt werden. Die Gasphase 107, die im Wesentlichen nicht umgesetztes Ethan sowie Ethylen und Kohlenmonoxid sowie gewisse Anteile von Kohlendioxid enthält wird sodann einer Verdichtung 40 auf ein benötigtes Druckniveau unterworfen, bei dem eine optionale Kohlendioxidentfernung 50, eine optionale weitere Reinigung 60 zur Entfernung von Spurenkomponenten und eine Hydroformylierung 70 erfolgen.

Eine Einspeisung von zusätzlich benötigtem Wasserstoff und/oder Kohlenmonoxid 108 kann dabei entsprechend dem Druckniveau vor der Verdichtung 40 bzw. vor einer entsprechenden Verdichterstufe oder aber auch nach der Verdichtung 40 erfolgen. In der Reinigung 60 können eine oder mehrere geeignete Verfahrenseinheiten zur Entfernung von Spurenkomponenten vorgesehen sein.

Ein in der Kohlendioxidentfernung 50 erhaltener Kohlendioxidstrom 109 kann zumindest teilweise als Reaktionseinsatz in die ODHE zurückgeführt werden.

Stromab der Hydroformylierung 70 wird in einer Trennung 80 von Kohlenwasserstoffen mit zwei und drei Kohlenstoffatomen ein an Propanal reicher Teilstrom 110 erhalten, der bedarfsweise weiteren Reaktionsschritten 90, welche insbesondere eine Hydrierung und/oder eine Dehydratisierung umfassen können, zugeführt werden kann. Der Kopfstrom 111 der genannten Trennung 80 ist insbesondere an Ethan angereichert und kann als Rückführstrom 112, enthaltend Kohlenwasserstoffe mit zwei Kohlenstoffatomen und leichtere Verbindungen, in die ODHE 10 zurückgeführt werden und dient dort wiederum als Reaktionseinsatz. Zur Vermeidung der Akkumulation von Inerten bzw. leichten Spurenkomponenten kann aus diesem Recyclestrom ein Purgestrom 113 abgeführt werden.

Der Rückführstrom 112 enthält in dieser Grundausführung neben Ethan aber auch nicht in der Hydroformylierung umgesetzte Anteile von Ethylen, Wasserstoff und/oder Kohlenmonoxid, die dann in der ODHE 10 zumindest teilweise zu weiteren Folgeprodukten, wie insbesondere Essigsäure, Wasser und Kohlendioxid, reagieren können. Die Trennung 80 von Kohlenwasserstoffen mit zwei und drei Kohlenstoffatomen ist dabei vorzugsweise nichtkryogen, z.B. als Extraktivdestillation, ausgeführt. Auf diese Weise kann in dem Verfahren 100 eine besonders vorteilhafte Verfahrensführung erreicht werden, die vollständig ohne kryogene Schritte auskommt.

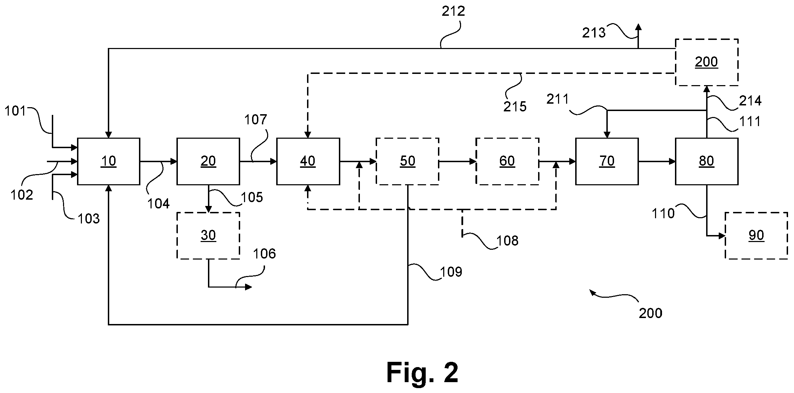

In Figur 2 ist ein Prozess gemäß einer weiteren bevorzugten Ausführungsform der vorliegenden Erfindung in Form eines schematischen Ablaufplans veranschaulicht und insgesamt mit 200 bezeichnet. Es gelten grundsätzlich die zuvor für Figur 1 und den dort gezeigten Prozess 100 beschriebenen Erläuterungen. Abweichungen sind im Folgenden erläutert.

Zur Bildung eines hier mit 212 bezeichneten Rückführstroms erfolgt hier zunächst eine Rückführung eines Teilstromes 211 des Kopfstroms 111 in die Hydroformylierung 70. Auf diese Weise kann der Gesamtumsatz in der Hydroformylierung 70 und somit die Propanalausbeute erhöht werden. Aufgrund des nach wie vor vorhandenen Ethans in ist die Menge dieser Rückführung jedoch technisch begrenzt. Zur weiteren Rückgewinnung von Wertkomponenten kann daher eine Wasserstoffabtrennung 200 durchlaufen werden, die mit einem Teilstrom 214 des Kopfstroms 111 gespeist wird, und die den dort enthaltenen Wasserstoff zumindest teilweise als einen wasserstoffangereicherten Teilstrom 215 zurückgewinnt. Dieser Teilstrom 215 kann dann entsprechend dem Druckniveau vor die Verdichtung 40 bzw. vor eine entsprechende Verdichterstufe zurückführt werden. Wasserstoff könnte ansonsten wie für das Verfahren 100 ausgeführt erwartungsgemäß in der ODHE zu Wasser verbrannt werden. Ein Purgestrom ist hier mit 213 bezeichnet.

Die Trennung 80 von Kohlenwasserstoffen mit zwei und drei Kohlenstoffatomen ist dabei auch in der in Figur 2 veranschaulichten Ausgestaltung vorzugsweise nichtkryogen, z.B. als Extraktivdestillation, ausgeführt und auf diese Weise kann in dem Verfahren 200 weiterhin eine besonders vorteilhafte Verfahrensführung erreicht werden, die vollständig ohne kryogene Schritte auskommt.

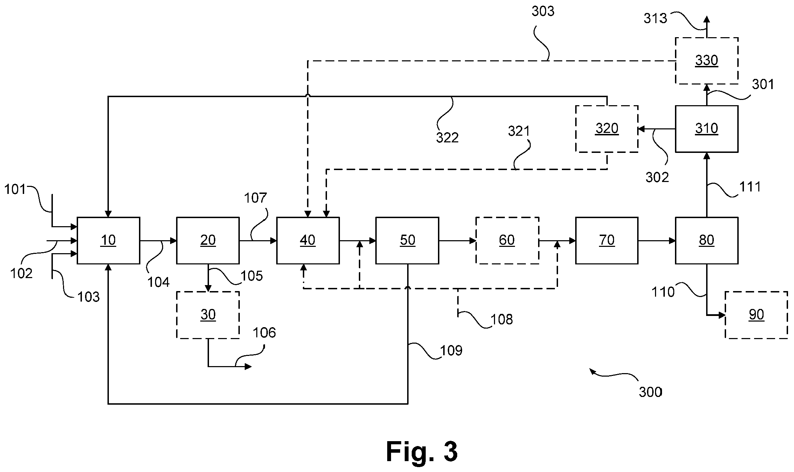

In Figur 3 ist ein Prozess gemäß einer weiteren bevorzugten Ausführungsform der vorliegenden Erfindung in Form eines schematischen Ablaufplans veranschaulicht und insgesamt mit 300 bezeichnet. Es gelten grundsätzlich die zuvor für Figur 1 und den dort veranschaulichten Prozess 100 beschriebenen Erläuterungen. Abweichungen davon sind im Folgenden erläutert.

Der Kopfstrom 111 der Trennung 80 von Kohlenwasserstoffen mit zwei und drei Kohlenstoffatomen wird in dem Verfahren 300 zunächst einer Abtrennung 310 von Kohlenwasserstoffen mit zwei Kohlenstoffatomen und leichteren Komponenten zugeführt. In dieser werden ein an den leichter siedenden Komponenten (insbesondere Kohlenmonoxid und Wasserstoff) angereicherter Teilstrom 301 und ein an Kohlenwasserstoffen mit zwei Kohlenstoffatomen (insbesondere Ethan und Ethylen) angereicherter Teilstrom 302 gebildet. Unter Ausschleusung eines Purgestroms 313 wird ein entsprechender Teilstrom 303 des Teilstroms 301 vorzugsweise vor die Verdichtung 40 bzw. vor eine entsprechende Verdichterstufe zurückführt. Somit können Wasserstoff und Kohlenmonoxid wieder in den Prozess 300 zurückgeführt werden. Hierdurch wird also die im Prozess 200 dargelegte Rückführung eines Teilstromes 211 in die Hydroformylierung 211 weiter optimiert.

Nur bei erheblichen Anteilen von weiteren Gasen im Teilstrom 301 kann eine weitere Aufreinigung 330 dieses Teilstromes vorgesehen sein, wie hier veranschaulicht, die dann den Teilstrom 303 in in an Kohlenmonoxid und/oder Wasserstoff angereicherter benötigter Qualität bereitstellt.

Zur weiteren Effizienzerhöhung kann der Teilstrom 302 einem Splitter 330 zugeführt werden. Ein hier gebildeter, an Ethylen angereicherter Teilstrom 321 wird dann auf möglichst hohem Druckniveau wieder in die Verdichtung 40 bzw. vor eine geeignete Verdichterstufe zurückgeführt und auf diese Weise wieder der Hydroformylierung 70 zugeführt. Ein weiterer, an Ethan angereicherter Teilstrom 322 wird der ODHE als Reaktionseinsatz zugeführt.

Bei dieser Ausführungsform kommen zwar kryogene Trennungen zum Einsatz, jedoch können alle Wertkomponenten in optimaler Weise innerhalb des Verfahrens 300 genutzt werden. Aufgrund der kryogenen Vefahrensschritte sind eine Kohlendioxidentfernung 50 und eine Trocknung (nicht dargestellt) bei dieser Variante jedoch obligatorisch.

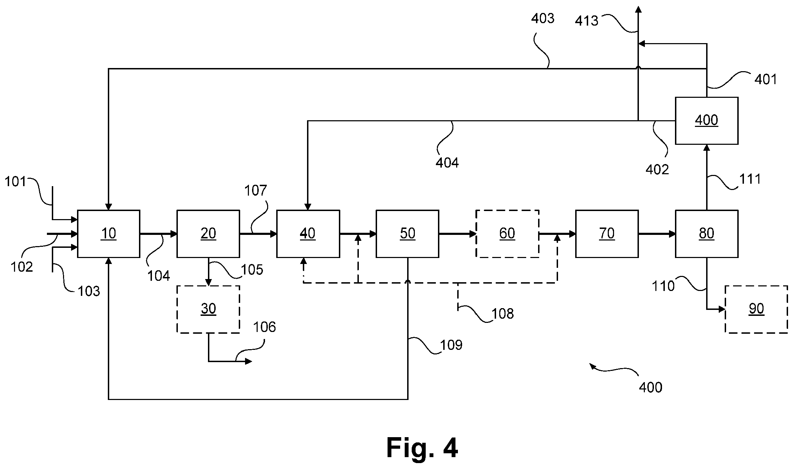

In Figur 4 ist ein Prozess gemäß einer weiteren bevorzugten Ausführungsform der vorliegenden Erfindung in Form eines schematischen Ablaufplans veranschaulicht und insgesamt mit 400 bezeichnet. Es gelten grundsätzlich die zuvor für Figur 3 und den dort veranschaulichten Prozess 300 beschriebenen Erläuterungen. Abweichungen sind im Folgenden erläutert.

Abweichend vom Verfahren 300 werden die Verfahrensschritte 310, 320 und 330 hier in einem Verfahrensschritt 400 zusammengefasst. Dies ist insbesondere dann möglich, wenn der Kopfstrom 111 der Trennung 80 außer Wasserstoff und Kohlenmonoxid keine nennenswerten Mengen an sonstigen leichten Komponenten (insbesondere Methan) enthält. In dem Verfahrensschritt 400, der grundsätzlich der Funktion eines Splitters entspricht, werden wiederum ein an Ethan angereicherter Teilstrom 401 sowie ein an Ethylen, Kohlenmonoxid und/oder Wasserstoff angereicherter Teilstrom 402 gebildet. Nach ggf. erfolgender Abzweigung eines zur Bildung eines Purgestroms 413 verwendeten Anteils wird der Teilstrom 401, nun mit 403 bezeichnet, der ODHE 10 als Reaktionseinsatz zugeführt. Der Teilstrom 402 wird, ebenfalls ggf. nach Abzweigung eines zur Bildung des Purgestroms 413 verwendeten Anteils, auf möglichst hohem Druckniveau wieder in die Verdichtung 40 bzw. vor eine geeignete Verdichterstufe zurückgeführt und auf diese Weise wieder der Hydroformylierung 70 zugeführt. Die Bildung des Purgestroms 413 bzw. Abzweigung entsprechender Teilströme, einzeln oder gemeinsam, erfolgt bei Bedarf.

Aufgrund dieser Prozessführung ist aber insbesondere keine besonders scharfe Trennung im Verfahrensschritt 400 notwendig, wie sie ansonsten üblicherweise in einem Splitter, in einer Dampfspaltanlage, angestrebt wird. Dies betrifft insbesondere das Kopfprodukt in Form des Stoffstroms 402, da eine im Teilstrom 404 enthaltene Restmenge an Ethan über den entsprechenden Recycle wieder in den Prozess zurückgeführt wird und die Hydroformylierung 70 als Inertkomponente durchläuft. Daher ist also eine hinreichende Abreicherung von Ethan in diesem Teilstrom 404 ausreichend und keine quantitative Abtrennung erforderlich.

Restmengen von Ethylen, Wasserstoff und/oder Kohlenmonoxid im Teilstrom 404 werden dann wie für Prozess 100 dargelegt in der ODHE zumindest teilweise zu weiteren Folgeprodukten, wie insbesondere Essigsäure, Wasser und Kohlendioxid, umgesetzt. Somit können auch hier gewisse Restgehalte der angegebenen Komponenten toleriert werden.

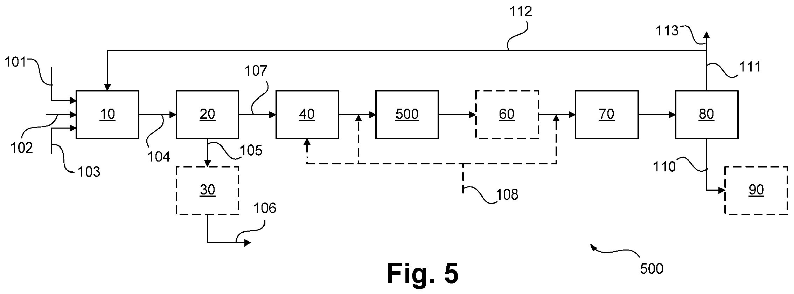

In Figur 5 ist ein Prozess gemäß einer weiteren bevorzugten Ausführungsform der vorliegenden Erfindung in Form eines schematischen Ablaufplans veranschaulicht und insgesamt mit 500 bezeichnet. Es gelten grundsätzlich die zuvor für Figur 1 und den dort veranschaulichten Prozess 100 beschriebenen Erläuterungen. Abweichungen sind im Folgenden erläutert.

Anstelle einer Kohlendioxidentfernung 50 ist hier eine Wassergasshift 500 stromauf der Hydroformylierung 70 vorgesehen. Hier wird im Prozessstrom enthaltenes Kohlendioxid mit Wasserstoff zu Kohlenmonoxid und Wasser umgesetzt. Vorteilhafterweise kann auf diese Weise eine genaue Anpassung des Verhältnisses Ethylen zu Kohlenmonoxid zu Wasserstoff entsprechend den Anforderungen der Hydroformylierung 70 erzielt werden. In der Wassergasshift 500 entstehendes Wasser kann bedarfsweise mittels Phasentrennung und/oder Trocknung (beide nicht dargestellt) abgetrennt bzw. entfernt werden. Ein expliziter Recycle 109 von Kohlendioxid entfällt und möglicherweise noch verbleibendes Kohlendioxid kann stattdessen mit dem Recylestrom 112 zur ODHE 10 zurückgeführt werden.

## Patentansprüche

1. Verfahren (100-500) zur Herstellung einer Zielverbindung, bei dem ein erstes Komponentengemisch unter Erhalt eines zweiten Komponentengemischs einer oxidativen Dehydrierung (10) unterworfen wird, und bei dem zumindest ein Teil des zweiten Komponentengemischs unter Erhalt eines dritten Komponentengemischs einer Hydroformylierung (20) unterworfen wird, wobei das erste Komponentengemisch ein Paraffin, das zweite Komponentengemisch ein in der oxidativen Dehydrierung (10) aus dem Paraffin gebildetes Olefin und das dritte Komponentengemisch eine in der Hydroformylierung (20) aus dem Olefin gebildete Folgeverbindung, die die Zielverbindung oder eine zu der Zielverbindung umsetzbare Vorläuferverbindung darstellt, enthalten, **dadurch gekennzeichnet, dass** das erste Komponentengemisch Sauerstoff in einem Sauerstoffgehalt und/oder Kohlendioxid in einem Kohlendioxidgehalt aufweist, und dass das zweite Komponentengemisch Kohlenmonoxid in einem Kohlenmonoxidgehalt und das Olefin in einem Olefingehalt aufweist, wobei der Kohlenmonoxidgehalt bei 5% bis 120% des Olefingehalts beträgt.

2. Verfahren (100-500) nach Anspruch 1, bei dem das Paraffin Ethan, das Olefin Ethylen und die Folgeverbindung Propanal darstellen.

3. Verfahren (100-500) nach Anspruch 2, bei dem das erste Komponentengemisch weniger als 25%, weniger als 10% oder weniger als 5% Kohlenmonoxid, 0,1 bis 50%, 5 bis 40% oder 10 bis 25% Kohlendioxid, 20 bis 85% oder 30 bis 60% Ethan, weniger als 20%, weniger als 10%, weniger als 5% oder weniger als 1% Ethylen, und 0,1 bis 30%, 2 bis 25% oder 5 - 20% Sauerstoff, jeweils auf Volumenbasis und ohne Berücksichtigung des durch Wasser gebildeten Anteils, enthält.

4. Verfahren (100-500) nach Anspruch 2 oder 3, bei das zweite Komponentengemisch 1% bis 60% oder 5% bis 40% Kohlenmonoxid, 0,1% bis 40%, 0,5% bis 25% oder 5 bis 20% Kohlendioxid, 0,1% bis 50%, 5% bis 40% oder 10% bis 30% Ethan, 10% bis 80% oder 30% bis 70% Ethylen und weniger als 10%, weniger als 5%, weniger als 1% oder weniger als 0,1% Sauerstoff, jeweils auf Volumenbasis und ohne Berücksichtigung des durch Wasser gebildeten Anteils, enthält.

5. Verfahren (100-500) nach Anspruch 3 oder 4, bei dem das erste Komponentengemisch 1% bis 95%, 5% bis 50% oder 10% bis 35% Wasser auf Volumenbasis und bezogen auf das gesamte erste Komponentengemisch, enthält.

6. Verfahren (100-500) nach einem der Ansprüche 2 bis 5, bei dem zumindest ein Teil des Propanals einer weiteren Umsetzung zu 1-Propanol unterworfen wird.

7. Verfahren (100-500) nach Anspruch 6, bei dem zuimdest ein Teil des 1-Propanols einer weiteren Umsetzung zu Propylen unterworfen wird und das Propylen in einer Reinheit von mindestens 80%, 90%, 95% oder 98% erhalten wird.

8. Verfahren (100-500) nach Anspruch 7, bei dem das erhaltene Propylen unter Erhalt eines Reinpropylenstromes zumindest teilweise mindestens einer weiteren Fraktionierung zugeführt wird.

9. Verfahren (100-500) nach einem der vorstehenden Ansprüche, bei dem dem ersten Komponentengemisch Kohlendioxid zugegeben wird, das aus dem zweiten Komponentengemisch oder dem dritten Komponentengemisch abgetrennt und/oder aus einer externen Quelle bereitgestellt wird.

10. Verfahren nach Anspruch 9, bei dem Kohlendioxid aus dem zweiten Komponentengemisch abgetrennt wird, wobei zumindest ein Teil des zweiten Komponentengemischs aus der oxidativen Dehydrierung (10) auf ein Druckniveau verdichtet wird, auf dem das Kohlendioxid aus dem zweiten Komponentengemisch abgetrennt und die Hydroformylierung (20) durchgeführt wird.

11. Verfahren (100-500) nach einem der vorstehenden Ansprüche, bei dem
(i) unter Verwendung zumindest eines Teils des zweiten und/oder dritten Komponentengemischs eine Ethan enthaltende Fraktion gebildet und in die oxidative Dehydrierung (10) zurückgeführt wird, und/oder
(ii) unter Verwendung zumindest eines Teils des dritten Komponentengemischs eine Ethylen, Wasserstoff und/oder Kohlenmonoxid enthaltende Fraktion gebildet und in die Hydroformylierung (20) zurückgeführt wird, und/oder
(iii) unter Verwendung zumindest eines Teils des dritten Komponentengemischs eine überwiegend oder ausschließlich Wasserstoff enthaltende Fraktion gebildet und in einer Hydrierung verwendet wird.

12. Verfahren (100-500) nach einem der vorstehenden Ansprüche, das stromab der oxidativen Dehydrierung (10) und stromab der Hydroformylierung (20) vollständig nicht-kryogen durchgeführt wird.

13. Verfahren (100-500) nach einem der vorstehenden Ansprüche, wobei stromauf der Hydroformylierung (20) Wasserstoff und/oder Kohlenmonoxid eingespeist und ein Verhältnis zwischen Wasserstoff und Kohlenmonoxid in einer Wassergasshift-Reaktion eingestellt wird.

14. Verfahren (100-500) nach einem der vorstehenden Ansprüche, bei dem ein zumindest die Elemente Mo, V, Nb und optional Te als Mischoxid enthaltender Katalysator in der oxidativen Dehydrierung verwendet wird.

15. Anlage, zur Herstellung einer Zielverbindung, die dafür eingerichtet ist, ein erstes Komponentengemisch unter Erhalt eines zweiten Komponentengemischs einer oxidativen Dehydrierung (10) zu unterwerfen zumindest einen Teil des zweiten Komponentengemischs unter Erhalt eines dritten Komponentengemischs einer Hydroformylierung (20) zu unterwerfen wird, wobei das erste Komponentengemisch ein Paraffin, das zweite Komponentengemisch ein in der oxidativen Dehydrierung (10) aus dem Paraffin gebildetes Olefin und das dritte Komponentengemisch eine in der Hydroformylierung (20) aus dem Olefin gebildete Folgeverbindung, die die Zielverbindung oder eine zu der Zielverbindung umsetzbare Vorläuferverbindung darstellt, enthalten, **dadurch gekennzeichnet,** die Anlage für einen Betrieb eingerichtet ist, der bewirkt, dass das erste Komponentengemisch Sauerstoff in einem Sauerstoffgehalt und/oder Kohlendioxid in einem Kohlendioxidgehalt aufweist, und dass das zweite Komponentengemisch Kohlenmonoxid in einem Kohlenmonoxidgehalt und das Olefin in einem Olefingehalt aufweist, wobei der Kohlenmonoxidgehalt bei 5% bis 120% des Olefingehalts beträgt.
